# FASCICULE DE BREVET EUROPEEN

(11) **EP 4 223 261 B1**
(45) Date de publication et mention de la délivrance du brevet: **13.08.2025**
(21) Numéro de dépôt: 23155698.6
(22) Date de dépôt: 08.02.2023
(51) Int. Cl.: A61F 5/28

(54) **DISPOSITIF DE SOUTIEN PELVIEN**
BECKENSTÜTZVORRICHTUNG
PELVIC SUPPORT DEVICE

(30) Priorité: 08.02.2022 FR 2201081
(43) Date de publication de la demande: 09.08.2023
(73) Titulaire: Femma, 91330 Yerres (FR)
(72) Inventeur: SANTOS PEREIRA, Emmanuelle, 91330 YERRES (FR)
(74) Mandataire: Icosa

(56) Documents cités:
- US-A- 3 116 736
- US-A1- 2015 034 074
- US-B1- 8 672 910
- US-B2- 9 452 100

## Description

La présente invention concerne un dispositif de soutien pelvien. Elle trouve notamment une application dans le cas de personnes nécessitant le port d'une culotte de compression de la zone pelvienne.

Tout au long de leur vie, les femmes peuvent être amenées à souffrir, de manière occasionnelle, voire permanente, de pathologies qui provoquent une sensation très incommodante, parfois invalidante, de pesanteur, de poids, au niveau du plancher pelvien.

Le plancher pelvien est constitué d'un ensemble de muscles allant de l'os pubien jusqu'à la colonne vertébrale en formant des anneaux autour de l'urètre, du vagin, et de l'anus. Ces muscles forment une sorte de hamac qui supporte le contenu abdominal et la colonne vertébrale, tout en permettant de maintenir de façon optimale les fonctions urinaires, rectales et sexuelles.

Parmi les pathologies rencontrées, on peut citer les varices vulvaires, les prolapsus génitaux-urinaires, le syndrome prémenstruel ou de douleurs menstruelles, le syndrome de congestion pelvienne, les oedèmes pelvi-vulvaires ou périnéaux (suite d'un accouchement ou d'une opération gynécologique).

Dans certain cas, la situation est tellement invalidante que les femmes ne peuvent plus se lever, ni marcher. Outre les douleurs physiques, ces situations peuvent donc engendrer des problèmes pratique importants avec impossibilité de travailler ou de s'occuper d'enfants par exemple, donc d'avoir une vie normale. Par ailleurs, les pathologies évoquées, si elles ne sont pas traitées de manière efficace, peuvent engendrer des séquelles : incontinences, descente d'organes, perte parfois irréversible de tonicité des veines vulvaires, douleurs pendant relations sexuelles, etc.

Il est donc important de pouvoir disposer dans ces situations d'une solution de soulagement efficace et confortable.

Les solutions médicamenteuse, par exemple à l'aide de médicaments de type veinotoniques, ou encore le port de bas de contention, sont inefficaces ou insuffisantes pour soulager la patiente de ses douleurs et agir sur la pesanteur qu'elle ressent.

On connaît des dispositifs prenant la forme d'une culotte de compression, tels que celui décrit dans le document US2015/0034074, qui sont destinés à soulager une femme souffrant de varices vulvaires. Le document US 9 452 100 B2 décrit un dispositif de soutien pelvien selon le préambule de la revendication 1.

D'une manière générale, un des inconvénients des dispositifs connus est qu'ils sont soit peu efficaces car n'appliquant pas suffisamment de pression sur la zone pelvienne, soit peu confortables en raison de la contention et de la pression qu'ils exercent .

Un des buts de l'invention est donc de résoudre notamment les problèmes précités. Ainsi, l'invention a notamment pour objectif de proposer un dispositif de soutien pelvien efficace, confortable et agréable à porter.

L'invention a ainsi pour objet un dispositif de soutien pelvien destiné à être porté par un utilisateur, comprenant une ceinture élastique présentant une partie avant et une partie arrière, deux sangles élastiques présentant chacune une extrémité avant reliée à la partie avant de la ceinture et une extrémité arrière, opposée à l'extrémité avant, reliée de manière amovible à la partie arrière de la ceinture. Les deux sangles se croisent en sorte de former avec la ceinture respectivement deux passages d'insertion des cuisses de l'utilisateur.

Le dispositif comprend également une bande de support pelvien destinée à être positionnée en sorte d'assurer un soutien au niveau de la zone pelvienne de l'utilisateur. Cette bande comprend une extrémité avant reliée à la partie avant de la ceinture et une extrémité arrière, opposée à l'extrémité avant. Les deux sangles se croisent en une zone de croisement située en regard de la bande, l'extrémité arrière de la bande étant reliée aux deux sangles en des zones de liaison arrière respectives de ces deux sangles situées entre la zone de croisement et les extrémités arrières respectives de ces deux sangles.

Suivant certains modes de réalisation, le dispositif comprend en outre une ou plusieurs des caractéristiques suivantes, prise(s) isolément ou suivant toutes les combinaisons techniquement possibles :
- la ceinture présente une valeur d'allongement manuel maximum sensiblement comprise entre 85% et 115%, de préférence sensiblement égale à 100%, et/ou une valeur de force requise pour un allongement de 30% sensiblement comprise entre 195 cN/cm et 255 cN/cm, de préférence sensiblement égale à 220 cN/cm ;
- la bande comprend au moins une première couche en matériau élastique et au moins une deuxième couche en matériau de type mousse alvéolée destinée à être en contact avec la zone pelvienne de l'utilisateur ;
- la deuxième couche comprend des premières fibres synthétiques élastiques, telles que des fibres d'élasthanne, est des deuxièmes fibres synthétiques, telles que des fibres en polyester ou en polyuréthane ;
- la deuxième couche comprend entre 93% et 97% de deuxièmes fibres, de préférence 95%, et entre 3% et 7% de premières fibres, de préférence 5% ;
- la première couche présente une valeur d'allongement manuel maximum sensiblement comprise entre 60% et 80%, de préférence sensiblement égale à 70%, et/ou une valeur de force requise pour un allongement de 30% sensiblement comprise entre 210 cN/cm et 290 cN/cm, de préférence sensiblement égale à 250 cN/cm ;
- la bande s'étend entre son extrémité avant et son extrémité arrière selon une première dimension, et présente une valeur d'allongement maximum dans la première direction sensiblement comprise entre 35% et 50%, de préférence sensiblement égale à 42%, et une valeur d'allongement maximum dans une deuxième direction, perpendiculaire à la première direction, sensiblement comprise entre 15% et 25%, de préférence sensiblement égale à 20% ;
- les sangles présentent une valeur d'allongement manuel maximum sensiblement comprise entre 60% et 80%, de préférence sensiblement égale à 70%, et/ou une valeur de force requise pour un allongement de 30% sensiblement comprise entre 350 cN/cm et 450 cN/cm, de préférence sensiblement égale à 400 cN/cm ;
- les extrémités avant des deux sangles sont reliées à la partie avant de la ceinture en des zones de liaison avant, et l'extrémité avant de la bande est reliée à la partie avant de la ceinture au moins en lesdites zones de liaison avant ;
- la longueur de chaque sangle est ajustable par des moyens d'ajustement disposés au niveau de l'extrémité ladite sangle et/ou au niveau d'une zone de liaison arrière de la ceinture en laquelle l'extrémité arrière de ladite sangle est reliée de manière amovible.

Ainsi, la structure du dispositif de l'invention le rend particulièrement efficace dans sa fonction de soutien pelvien, assurant une bonne compression pour soulager l'utilisateur. Dans le même temps, cette structure rend le dispositif confortable.

Précisément, la structure de la bande de soutien pelvien et la façon dont elle est reliée aux sangles et à la ceinture, permettent d'obtenir un résultat optimum en termes de soutien et de confort.

La fonction de soutien, assurée par la bande de soutien pelvien, est renforcée par la zone de croisement des sangles, en regard de la bande.

En particulier, les caractéristiques d'élasticité de la ceinture ont été déterminées après de nombreux essais, pour offrir le meilleur compromis entre la compression requise au niveau pelvien et le confort d'utilisation. Il en va de même des caractéristiques des sangles, et de la bande de support pelvien.

Le dispositif de l'invention prend la forme d'une culotte de contention qui soulage instantanément les femmes souffrant des pathologies précitées grâce à la compression, au soutien et au confort qu'elle procure à tout le plancher pelvien.

Les caractéristiques et avantages de l'invention apparaitront à la lecture de la description qui va suivre, donnée uniquement à titre d'exemple, et non limitative, en référence aux figures annexées suivantes :
- figure 1 : représentation schématique d'un exemple de dispositif de l'invention, vue par l'avant ;
- figure 2 : représentation schématique d'un exemple de dispositif de l'invention, vue par l'arrière.

Comme on peut le voir dans les exemples représentés sur les figures, le dispositif prend la forme d'une culotte. Ce dispositif comprend plus précisément une ceinture 1 élastique, deux sangles 2, 3 également élastiques et une bande 4 de support pelvien.

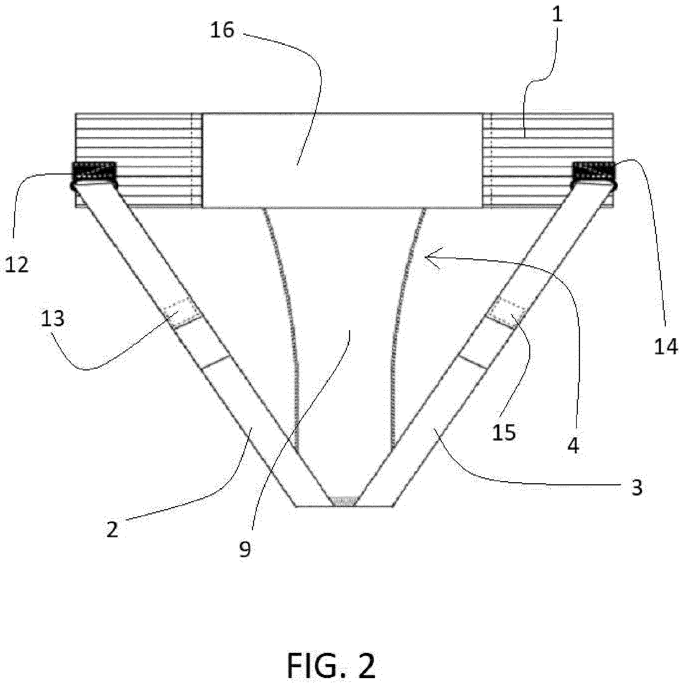

La ceinture 1 présente une partie avant, visible sur la figure 2, et une partie arrière opposée à la partie avant, visible sur la figure 2. La partie arrière est donc destinée à être positionnée à l'arrière de l'utilisateur, du côté du dos, alors que la partie avant est destinée à être positionnée à l'avant de l'utilisateur, du côté du ventre.

Chacun des deux sangles 2, 3 présente une extrémité avant, et une extrémité arrière opposée à l'extrémité avant. Les extrémités avant respectives des deux sangles 2, 3 sont reliée à la partie avant de la ceinture 1. Par ailleurs, les parties arrières respectives de ces deux sangles 2, 3 sont reliée de manière amovible à la partie arrière de la ceinture 1.

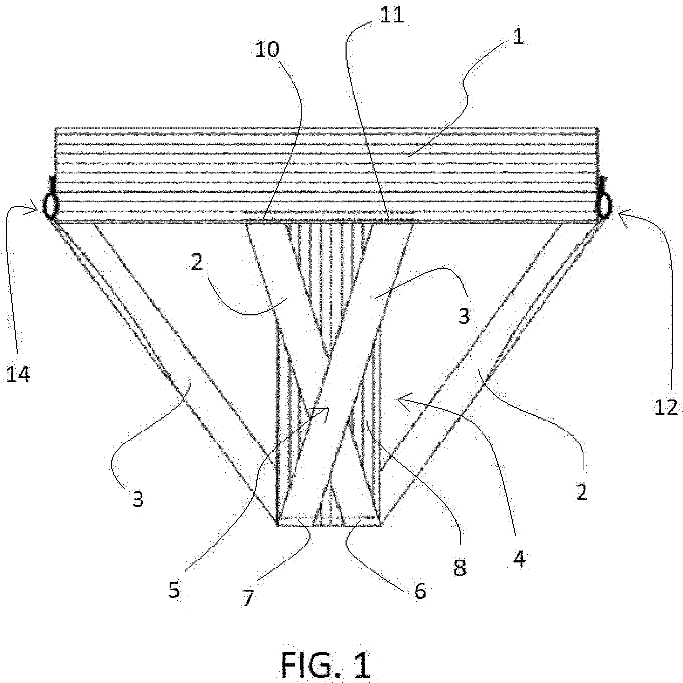

Comme on le voit plus précisément sur la figure 1, ces deux sangles 2, 3 se croisent en une zone de croisement 5, en sorte de former avec la ceinture 1 respectivement deux passages d'insertion des cuisses de l'utilisateur.

La bande 4 est destinée à être positionnée en sorte d'assurer un soutien au niveau d'au moins une partie de la zone pelvienne de l'utilisateur. Précisément, cette bande 4 comprend une extrémité avant et une extrémité arrière, opposée à l'extrémité avant. L'extrémité avant est reliée à la partie avant de la ceinture 1. Il n'y a donc pas d'espace entre la bande 4 et la partie avant de la ceinture 1, ce qui permet à la bande 4 d'envelopper correctement la partie avant de la zone pelvienne et d'apporter le soutien recherché, tout en offrant un bon confort d'utilisation. Par ailleurs, l'extrémité arrière est reliée aux deux sangles 2, 3 en des zones de liaison arrière 6, 7 respectives de ces deux sangles 2, 3, comme on peut le voir plus précisément sur la figure 1.

Ainsi, la partie arrière de la bande 4 est reliée à la zone 6 de la sangle 2, et à la zone 7 de la sangle 3. Précisément, ces zones de liaison arrières 6, 7 des deux sangles respectives 2, 3 sont situées entre la zone de croisement 5 et les extrémités arrières respectives de ces deux sangles 2, 3.

La bande 4 peut s'étendre plus ou moins loin au-delà de la zone de croisement 5, en direction de la partie arrière de la ceinture 1, en fonction de l'utilisateur et/ou de la portion de la zone pelvienne en laquelle l'effet de soutien ou de compression est recherché. Notamment, pour un effet de soutien recherché dans la zone anale, en cas d'hémorroïdes par exemple, la bande 4 peut être conçue pour s'étendre en direction de la partie arrière de la ceinture 1 suffisamment pour être en contact avec la zone anale.

Par ailleurs, comme on peut le voir sur la figure 1, la zone de croisement 5 est située en regard de la bande 4. Ainsi, la zone de croisement 5 des sangles 2, 3 contribue également au soutien pelvien.

La ceinture 1 présente une valeur d'allongement manuel maximum sensiblement comprise entre 85% et 115%, de préférence sensiblement égale à 100%.

Alternativement ou en combinaison, la ceinture 1 présente une valeur de force requise pour un allongement de 30% sensiblement comprise entre 195 cN/cm et 255 cN/cm, de préférence sensiblement égale à 220 cN/cm.

La valeur d'allongement maximum, tout comme la valeur de force requise pour un allongement de 30%, peuvent être mesurées par des moyens classiques de mesure connus de l'homme du métier.

Les intervalles précités, en particulier les valeurs préférentielles précitées, permettent d'obtenir le meilleur compromis entre la compression requise au niveau pelvien, donc l'efficacité du soutien pelvien, et le confort d'utilisation.

En ce qui concerne la bande 4, elle comprend au moins une première couche 7 en matériau élastique, visible sur la figure 1, et au moins une deuxième couche 9 en matériau de type mousse alvéolée, destinée à être en contact avec la zone pelvienne de l'utilisateur et visible sur la figure 2.

De préférence, la deuxième couche 9 comprend un premier type de fibres synthétiques élastiques, et un deuxième type de fibres synthétiques. Les fibre du premier type peuvent être par exemple en élasthanne. Par ailleurs, les fibres du deuxième type peuvent être par exemple en polyester ou en polyuréthane.

Il a été déterminé que les meilleurs résultats en termes de confort et d'efficacité de soutien pelvien étaient obtenus avec une première couche 8 et deuxième couche 9 qui présentent les caractéristiques spécifiques qui suivent.

En ce qui concerne la deuxième couche 9, celle-ci comprend entre 93% et 97% de deuxièmes fibres (par exemple en élasthanne), de préférence 95%, et entre 3% et 7% de premières fibres (par exemple en polyester ou en polyuréthane), de préférence 5%.

En ce qui concerne la première couche 8, celle-ci présente une valeur d'allongement manuel maximum sensiblement comprise entre 60% et 80%, de préférence sensiblement égale à 70%. Alternativement, ou en combinaison, cette première couche 8 présente par ailleurs une valeur de force requise pour un allongement de 30% sensiblement comprise entre 210 cN/cm et 290 cN/cm, de préférence sensiblement égale à 250 cN/cm.

En outre, la bande 4 s'étend entre son extrémité avant et son extrémité arrière selon une première dimension et une deuxième direction perpendiculaire à la première direction. Dans l'exemple représenté sur les figures, la forme d'ensemble de la bande 4 est sensiblement triangulaire ou trapézoïdale. La première dimension correspond à la plus grande dimension (la longueur), et la deuxième dimension correspond à la dimension perpendiculaire à cette plus grande dimension (la largeur),

Pour de résultats optimums en termes de confort et d'efficacité de soutien pelvien, il a été déterminé que les meilleurs résultats étaient obtenus avec une bande 4 configurée pour présenter une valeur d'allongement maximum dans la première direction sensiblement comprise entre 35% et 50%, de préférence sensiblement égale à 42%, et une valeur d'allongement maximum dans la deuxième direction, sensiblement comprise entre 15% et 25%, de préférence sensiblement égale à 20%.

Concernant les sangles 2, 3, celles-ci présentent une valeur d'allongement manuel maximum sensiblement comprise entre 60% et 80%, de préférence sensiblement égale à 70%. Par ailleurs, alternativement ou en combinaison, ces sangles 2, 3 présentent une valeur de force requise pour un allongement de 30% sensiblement comprise entre 350 cN/cm et 450 cN/cm, de préférence sensiblement égale à 400 cN/cm.

Là encore, les intervalles de valeurs présentés ci-dessus, en particulier les valeurs préférentielles, pour les sangles 2, 3, permettent d'obtenir une solution optimum en termes de confort et d'efficacité de soutien pelvien.

Sur la figure 1, on peut voir que les extrémités avant de chacune des deux sangles 2, 3 sont reliées à la partie avant de la ceinture 1. Précisément, la sangle 2 est reliée à la partie avant de la ceinture 1 en une zone de liaison avant 10, et la sangle 3 est reliée à la partie avant de la ceinture 1 en une zone de liaison avant 11.

L'extrémité avant de la bande 4 est quant à elle reliée à la partie avant de la ceinture 1 en une zone qui correspond aux, ou qui comprend les zones de liaison avant 10, 11.

Comme on peut le voir sur les figures, on prévoit que la longueur de chaque sangle 2, 3 puisse être ajustée ; Pour ce faire, des moyens d'ajustement 12 à 15 sont prévus, disposés au niveau des extrémités de chacune des sangles 2, 3, et/ou au niveau d'une zone de liaison arrière de la ceinture 1 en laquelle l'extrémité arrière de la sangle 2, 3 correspondante est reliée de manière amovible.

Dans l'exemple représenté sur les figures, un anneau 12 est fixé en la zone de liaison arrière de la ceinture 1 en laquelle l'extrémité arrière de la sangle 2 est reliée de manière amovible. Cette extrémité arrière de la sangle 2 passe dans l'anneau 12, se replie sur une longueur appropriée et vient se fixer sur un élément de fixation 13 positionné sur la sangle 2 (par exemple liaison de type boucle - crochet). De même, un anneau 14 est fixé en la zone de liaison arrière de la ceinture 1 en laquelle l'extrémité arrière de la sangle 3 est reliée de manière amovible. Cette extrémité arrière de la sangle 3 passe dans l'anneau 14, se replie sur une longueur appropriée et vient se fixer sur un élément de fixation 15 positionné sur la sangle 3 (par exemple liaison de type boucle - crochet).

Comme on peut le voir sur la figure 2, on peut prévoir une zone de renfort 16 sur la partie arrière de la ceinture 1, qui a pour effet d'apporter un maintien plus important au niveau du bas du dos de l'utilisateur.

La présente description est donnée à titre d'exemple et n'est pas limitative de l'invention. En particulier, l'invention ne se limite pas à un dispositif présentant des dimensions relatives particulières telles que celles représentées sur les figures.

## Revendications

1. Dispositif de soutien pelvien destiné à être porté par un utilisateur, comprenant une ceinture (1) élastique présentant une partie avant et une partie arrière, **caractérisé en ce qu'**il comprend deux sangles (2, 3) élastiques présentant chacune une extrémité avant reliée à la partie avant de la ceinture (1) et une extrémité arrière, opposée à l'extrémité avant, reliée de manière amovible à la partie arrière de la ceinture (1), les deux sangles (2, 3) se croisant en sorte de former avec la ceinture (1) respectivement deux passages d'insertion des cuisses de l'utilisateur, et une bande (4) de support pelvien destinée à être positionnée en sorte d'assurer un soutien au niveau de la zone pelvienne de l'utilisateur, la bande (4) comprenant une extrémité avant reliée à la partie avant de la ceinture (1) et une extrémité arrière, opposée à l'extrémité avant, les deux sangles (2, 3) se croisant en une zone de croisement (5) située en regard de la bande (4), l'extrémité arrière de la bande (4) étant reliée aux deux sangles (2, 3) en des zones de liaison arrière (6, 7) respectives de ces deux sangles (2, 3) situées entre la zone de croisement (5) et les extrémités arrières respectives de ces deux sangles (2, 3).

2. Dispositif selon la revendication 1, **caractérisé en ce que** la ceinture (1) présente une valeur d'allongement manuel maximum sensiblement comprise entre 85% et 115%, de préférence sensiblement égale à 100%, et/ou une valeur de force requise pour un allongement de 30% sensiblement comprise entre 195 cN/cm et 255 cN/cm, de préférence sensiblement égale à 220 cN/cm.

3. Dispositif selon l'une quelconque des revendications 1 et 2, **caractérisé en ce que** la bande (4) comprend au moins une première couche (7) en matériau élastique et au moins une deuxième couche (9) en matériau de type mousse alvéolée destinée à être en contact avec la zone pelvienne de l'utilisateur.

4. Dispositif selon la revendication 3, **caractérisé en ce que** la deuxième couche (9) comprend des premières fibres synthétiques élastiques, telles que des fibres d'élasthanne, est des deuxièmes fibres synthétiques, telles que des fibres en polyester ou en polyuréthane.

5. Dispositif selon l'une quelconque des revendications 3 et 4, **caractérisé en ce que** la deuxième couche (9) comprend entre 93% et 97% de deuxièmes fibres, de préférence 95%, et entre 3% et 7% de premières fibres, de préférence 5%.

6. Dispositif selon l'une quelconque des revendications 3 à 5, **caractérisé en ce que** la première couche (8) présente une valeur d'allongement manuel maximum sensiblement comprise entre 60% et 80%, de préférence sensiblement égale à 70%, et/ou une valeur de force requise pour un allongement de 30% sensiblement comprise entre 210 cN/cm et 290 cN/cm, de préférence sensiblement égale à 250 cN/cm.

7. Dispositif selon l'une quelconque des revendications 1 à 6, **caractérisé en ce que** la bande (4) s'étend entre son extrémité avant et son extrémité arrière selon une première dimension, et présente une valeur d'allongement maximum dans la première direction sensiblement comprise entre 35% et 50%, de préférence sensiblement égale à 42%, et une valeur d'allongement maximum dans une deuxième direction, perpendiculaire à la première direction, sensiblement comprise entre 15% et 25%, de préférence sensiblement égale à 20%.

8. Dispositif selon l'une quelconque des revendication 1 à 7, **caractérisé en ce que** les sangles (2, 3) présentent une valeur d'allongement manuel maximum sensiblement comprise entre 60% et 80%, de préférence sensiblement égale à 70%, et/ou une valeur de force requise pour un allongement de 30% sensiblement comprise entre 350 cN/cm et 450 cN/cm, de préférence sensiblement égale à 400 cN/cm.

9. Dispositif selon l'une quelconque des revendication 1 à 8, **caractérisé en ce que** les extrémités avant des deux sangles (2, 3) sont reliées à la partie avant de la ceinture (1) en des zones de liaison avant (10, 11), et **en ce que** l'extrémité avant de la bande (4) est reliée à la partie avant de la ceinture (1) au moins en lesdites zones de liaison avant (10, 11).

10. Dispositif selon l'une quelconque des revendications 1 à 9, **caractérisé en ce que** la longueur de chaque sangle (2, 3) est ajustable par des moyens d'ajustement (12 à 15) disposés au niveau de l'extrémité ladite sangle (2, 3) et/ou au niveau d'une zone de liaison arrière de la ceinture (1) en laquelle l'extrémité arrière de ladite sangle (2, 3) est reliée de manière amovible.

## Patentansprüche

1. Beckenstützvorrichtung zum Tragen durch einen Benutzer, umfassend einen elastischen Gürtel (1) mit einem Vorderteil und einem Hinterteil, **dadurch gekennzeichnet, dass** sie zwei elastische Gurte (2, 3) umfasst, die jeweils ein mit dem Vorderteil des Gürtels (1) verbundenes vorderes Ende und ein hinteres Ende aufweisen, das dem vorderen Ende gegenüberliegt und abnehmbar mit dem Hinterteil des Gürtels (1) verbunden ist, wobei sich die beiden Gurte (2, 3) so überschneiden, dass sie mit dem Gürtel (1) jeweils zwei Einführöffnungen für die Oberschenkel des Benutzers bilden, und ein Beckenstützband (4), das so positioniert werden soll, dass es eine Stütze im Beckenbereich des Benutzers bietet, wobei das Band (4) ein vorderes Ende umfasst, das mit dem Vorderteil des Gürtels (1) verbunden ist, und ein hinteres Ende, das dem vorderen Ende gegenüberliegt, wobei sich die beiden Gurte (2, 3) in einem Kreuzungsbereich (5) gegenüber dem Band (4) kreuzen, wobei das hintere Ende des Bandes (4) mit den beiden Gurten (2, 3) in jeweils hinteren Verbindungsbereichen (6, 7) dieser beiden Gurte (2, 3) verbunden ist, die sich zwischen dem Kreuzungsbereich (5) und den jeweiligen hinteren Enden dieser beiden Gurte (2, 3) befinden.

2. Vorrichtung nach Anspruch 1, **dadurch gekennzeichnet, dass** der Gürtel (1) einen maximalen manuellen Dehnungswert im Wesentlichen zwischen 85 % und 115 %, vorzugsweise im Wesentlichen gleich 100 %, und/oder einen für eine Dehnung von 30 % erforderlichen Kraftwert im Wesentlichen zwischen 195 cN/cm und 255 cN/cm, vorzugsweise im Wesentlichen gleich 220 cN/cm, aufweist.

3. Vorrichtung nach einem der Ansprüche 1 und 2, **dadurch gekennzeichnet, dass** das Band (4) mindestens eine erste Schicht (7) aus elastischem Material und mindestens eine zweite Schicht (9) aus wabenförmigem Schaumstoff umfasst, die dazu bestimmt ist, mit dem Beckenbereich des Benutzers in Kontakt zu sein.

4. Vorrichtung nach Anspruch 3, **dadurch gekennzeichnet, dass** die zweite Schicht (9) erste elastische synthetische Fasern, wie Elastanfasern, und zweite synthetische Fasern, wie Polyester- oder Polyurethanfasern, umfasst.

5. Vorrichtung nach einem der Ansprüche 3 und 4, **dadurch gekennzeichnet, dass** die zweite Schicht (9) zwischen 93 % und 97 % zweite Fasern, vorzugsweise 95 %, und zwischen 3 % und 7 % erste Fasern, vorzugsweise 5 %, umfasst.

6. Vorrichtung nach einem der Ansprüche 3 bis 5, **dadurch gekennzeichnet, dass** die erste Schicht (8) einen maximalen manuellen Dehnungswert im Wesentlichen zwischen 60 % und 80 %, vorzugsweise im Wesentlichen gleich 70 %, und/oder einen für eine Dehnung von 30 % erforderlichen Kraftwert im Wesentlichen zwischen 210 cN/cm und 290 cN/cm, vorzugsweise im Wesentlichen gleich 250 cN/cm, aufweist.

7. Vorrichtung nach einem der Ansprüche 1 bis 6, **dadurch gekennzeichnet, dass** sich das Band (4) zwischen seinem vorderen Ende und seinem hinteren Ende nach einer ersten Abmessung erstreckt und einen maximalen Dehnungswert in der ersten Richtung von im Wesentlichen zwischen 35 % und 50 %, vorzugsweise im Wesentlichen gleich 42 %, und einen maximalen Dehnungswert in einer zweiten Richtung, die senkrecht zur ersten Richtung verläuft, von im Wesentlichen zwischen 15 % und 25 %, vorzugsweise im Wesentlichen gleich 20 %, aufweist.

8. Vorrichtung nach einem der Ansprüche 1 bis 7, **dadurch gekennzeichnet, dass** die Gurte (2, 3) einen maximalen manuellen Dehnungswert im Wesentlichen zwischen 60 % und 80 %, vorzugsweise im Wesentlichen gleich 70 %, und/oder einen für eine Dehnung von 30 % erforderlichen Kraftwert im Wesentlichen zwischen 350 cN/cm und 450 cN/cm, vorzugsweise im Wesentlichen gleich 400 cN/cm, aufweisen.

9. Vorrichtung nach einem der Ansprüche 1 bis 8, **dadurch gekennzeichnet, dass** die vorderen Enden der beiden Gurte (2, 3) in vorderen Verbindungsbereichen (10, 11) mit dem Vorderteil des Gürtels (1) verbunden sind, und dass das vordere Ende des Bandes (4) mindestens in den vorderen Verbindungsbereichen (10, 11) mit dem Vorderteil des Gürtels (1) verbunden ist.

10. Vorrichtung nach einem der Ansprüche 1 bis 9, **dadurch gekennzeichnet, dass** die Länge jedes Gurts (2, 3) durch Einstellmittel (12 bis 15) verstellbar ist, die am Ende des Gurts (2, 3) und/oder an einem hinteren Verbindungsbereich des Gürtels (1) angeordnet sind, an dem das hintere Ende des Gurts (2, 3) lösbar verbunden ist.

## Claims

1. A pelvic support device intended to be worn by a user, comprising an elastic belt (1) having a front portion and a rear portion, **characterized in that** said device comprises two elastic straps (2, 3) each having a front end connected to the front portion of the belt (1) and a rear end, opposite the front end, removably connected to the rear portion of the belt (1), the two straps (2, 3) crossing so as to form with the belt (1) respectively two insertion passages of the user's thighs, and a pelvic support band (4) intended to be positioned so as to provide support for the user's pelvic area, the band (4) comprising a front end connected to the front part of the belt (1) and a rear end, opposite the front end, the two straps (2, 3) crossing each other in a crossing zone (5) located opposite the band (4), the rear end of the band (4) being connected to the two straps (2, 3) in respective rear connection zones (6, 7) of these two straps (2, 3) located between the crossing zone (5) and the respective rear ends of these two straps (2, 3)

2. Device according to claim 1, **characterized in that** the belt (1) has a maximum manual elongation value substantially between 85% and 115%, preferably substantially equal to 100%, and/or a force value required for 30% elongation substantially between 195 cN/cm and 255 cN/cm, preferably substantially equal to 220 cN/cm.

3. Device according to any one of claims 1 and 2, **characterized in that** the band (4) comprises at least one first layer (7) of elastic material and at least one second layer (9) of cellular foam-type material intended to be in contact with the user's pelvic area.

4. A device according to claim 3, **characterized in that** the second layer (9) comprises first elastic synthetic fibers, such as elastane fibers, and second synthetic fibers, such as polyester or polyurethane fibers.

5. A device according to any one of claims 3 and 4, **characterized in that** the second layer (9) comprises between 93% and 97% second fibers, preferably 95%, and between 3% and 7% first fibers, preferably 5%.

6. Device according to any one of claims 3 to 5, **characterized in that** the first layer (8) has a maximum manual elongation value substantially between 60% and 80%, preferably substantially equal to 70%, and/or a force value required for 30% elongation substantially between 210 cN/cm and 290 cN/cm, preferably substantially equal to 250 cN/cm.

7. Device according to any one of claims 1 to 6, **characterized in that** the strip (4) extends between its front end and its rear end along a first dimension, and has a maximum elongation value in the first direction substantially between 35% and 50%, preferably substantially equal to 42%, and a maximum elongation value in a second direction, perpendicular to the first direction, substantially between 15% and 25%, preferably substantially equal to 20%.

8. Device according to any one of claims 1 to 7, **characterized in that** the straps (2, 3) have a maximum manual elongation value substantially between 60% and 80%, preferably substantially equal to 70%, and/or a force value required for an elongation of 30% substantially between 350 cN/cm and 450 cN/cm, preferably substantially equal to 400 cN/cm.

9. Device according to any one of claims 1 to 8, **characterized in that** the front ends of the two straps (2, 3) are connected to the front part of the belt (1) at front connection areas (10, 11), and **in that** the front end of the band (4) is connected to the front part of the belt (1) at least at said front connection areas (10, 11).

10. A device according to any one of claims 1 to 9, **characterized in that** the length of each strap (2, 3) is adjustable by adjustment means (12 to 15) arranged at the end of said strap (2, 3) and/or at a rear connection area of the belt (1) to which the rear end of said strap (2, 3) is detachably connected.
